# EUROPEAN PATENT SPECIFICATION

(11) **EP 1 583 474 B1**
(45) Date of publication and mention of the grant of the patent: **07.05.2008**
(21) Application number: 04701866.8
(22) Date of filing: 14.01.2004
(51) Int. Cl.: A61B 17/00

(54) **DEVICE FOR REMOVAL OF BLOOD VESSELS OUT OF THE HUMAN BODY**
VORRICHTUNG ZUR ENTFERNUNG EINES BLUTGEFÄSSES AUS DEM KÖRPER
DISPOSITIF PERMETTANT DE RETIRER DES VAISSEAUX SANGUINS DU CORPS HUMAIN

(30) Priority: 17.01.2003 BE 200300038
(43) Date of publication of application: 12.10.2005
(73) Proprietor: Lesors B.V.B.A., 2440 Geel (BE)
(72) Inventor: OPHEIDE, Joseph, B-2440 Geel (BE); REHER, Stefan, B-2400 Mol (BE)
(74) Representative: Overath, Philippe
(86) International application number: PCT/BE2004/000008
(87) International publication number: WO 2004/064884

(56) References cited:
- WO-A-96/20646
- DE-A- 19 817 979
- DE-C- 342 110
- FR-A- 2 727 617
- GB-A- 2 082 459
- GB-A- 2 194 736

## Description

### TECHNICAL FIELD OF THE INVENTION

The present invention relates to the removal of blood vessels such as veins out of a human body and in particular to a device for the removal of a part of the superficial vein known as Vena Saphena Magna (VSM). The device according to the present invention also provides improved means for the harvesting of vessels which can be readily used for bypass purposes.

### BACKGROUND OF THE INVENTION

The blood circulation in the human body consists of arteries that carry the blood from the heart to different parts of the body, such as the limbs and veins, returning blood from the limb to the heart. Veins have internal valves to prevent from blood reflux. Contraction of the limb muscles forces the blood to the heart. These valves are important to prevent blood reflux to the limbs, such as the lower part of the legs, when they are at rest.

Defective or insufficient activity of the venous valves increases the blood pressure in the veins, resulting in dilatation and deformation. This venous deformation is known as varices and is mostly visible in the superficial veins of the subcutaneous tissue of the legs. The blood reflux from the legs is also performed by veins, located deeper under the skin.

In order to prevent complications in case of varices, the superficial vein or Vena Saphena Magna (VSM) is usually removed from the leg. Previously, this VSM was extirpated from the groin to the ankle, but at present only the part between the groin and the knee is removed to prevent nerve injury of a small cutaneous nerve located at the knee level. The usual technique to perform this surgical operation is called VSM 'stripping'.

In normal circumstances the blood returns from the feet to the heart due to the muscle pump. On the way up this vein gets different side branches until the vein itself flows into the deep venous system, which is positioned deeper in the groin, and which carries the blood back to the heart.

In order to proceed with this surgical operation, the outflow of the VSM in the deep system is dissected via a small incision in the groin. The deep system remains untouched and the VSM is stripped up to the knee level. To perform this procedure a lot of options are available.

For clarity reasons and in order to improve the general understanding of the following description, it has to be mentioned that 'distal' means away from the heart, and 'proximal' means in a direction towards the heart.

A commonly used technique is a plastic rope or iron rod being pushed into the vessel from the groin down to the knee level. Usually, a second incision is performed at knee level to pull out the vein. The rope or rod is pulled out at knee level and a small olive shaped object is attached to the rope end near the knee. The rope is then pulled from the knee towards the groin and because the 'olive' is too thick to pass into the vessel, the vein remains tied up against the olive and the vessel is pulled or stripped out at the of the upper leg at the groin level.

Because this method causes a lot of trauma by passing through the subcutaneous tissue, there are new alternatives of this technique without the use of an olive. The vessel is now usually attached to one end of the rope or iron rod, either in the groin (the proximal end of the vein) or at a second incision at the knee (distal part of the vein).

Because the rod and the rope are located inside the vein, that has to be pulled out, this vein will turn inside out by pulling the rod or rope and the stripping will cause less trauma than the previous technique. Such a method of pulling the vein inside out is called stripping by invagination. This method creates problems too because the vessel is torn out on its own, and because these vessels are very fragile, the vessel frequently tears up during the stripping operation.

The medical literature is often mentioning this procedure can take place under local anesthesia, contrary to the technique using the olive, but it remains painful because traction is always necessary and the vessel has to be freed from the surrounding tissues as well as from the side branches during the pull-out.

This technique is disclosed in the patent US 6,030,396, in which the pulling, inside out, of the vein is facilitated by the local injection, via a catheter, of a liquid under pressure to expand the vessel wall at the stripping side in order to make the stripping easier.

A further improvement of the technique is described in the document WO 0042918. This patent discloses a device for the stripping of veins by means of a catheter by which the distal end is provided with cutting devices. If the rod or catheter is brought inside the vein from the groin till the distal end of the rod arrives at the knee, the vein can be cut with internal cutting devices and perform a stripping without making a second skin incision at knee level.

GB-A-2 082 459 discloses a vein removal device having the features of the preamble of claim 1.

Beside the trauma caused by the stripping techniques another problem appears relating to the side branches of the VSM which are torn off from the VSM causing heavy blood loss and whereby the extirpated vessel, in most cases, is not suitable for reuse as a bypass for patients in need of a peripheral or cardiac bypass.

Besides the mechanical techniques mentioned above there are a number of other techniques by which the vessel is destroyed by freezing or burning techniques. These techniques are not considered in the present description because they are not frequently practiced and because they do not allow the reuse of the vein.

### SUMMARY OF THE INVENTION

In order to avoid the several drawbacks explained hereabove, it is the purpose of this invention to propose a device by which the vein, which has to be removed, is completely isolated from the surrounding subcutaneous tissue before the vessel is being removed from the body without performing a stripping operation.

Another purpose of this invention is to propose a device that makes it possible to achieve vessel harvesting by which the vessels, which are to be removed from the body, remain in such good condition that they can readily be used for bypass purposes.

This purpose is achieved because the device is, essentially, composed of two parts: a first part being a rod capable of being inserted inside the blood vessel, and a second part, consisting of a tube configured to be pushed over the outside surface of the vessel to isolate this vessel completely from the surrounding tissues, as claimed in claim 1 with preferred embodiments in the dependent claims.

In a preferred embodiment of the invention, the rigid rod, having a proximal end and a distal end. The proximal end being provided with a corkscrew like curl and an adjacent section having a roughened surface. The distal end being provided with a slightly bended, non traumatic shaped extremity and, in its close proximity, a substantially round or oval shaped broadened portion of the rod.

The tube, also having a proximal and a distal end, the distal end being provided with at least one, preferably two to four, longitudinal V-shaped slots, leaving between them equally spaced conical fingers extending towards the distal end of the tube.

By means of such device, it becomes possible to push the rod into the proximal end of the vessel that has to be removed; the distal end of the rod is to be pushed inside as far as the knee level. This operation straightens the vessel (which is often twisted and tortuous). The proximal part of the vessel (VSM) is tied up on the rough surface of the rigid rod.
The tube is then pulled over the vessel with an alternate rotational movement during which the vein is isolated from the surrounding tissue by means of the longitudinal slots and the protruding fingers (vibration means could also be applied on the tube if appropriate during this movement). This handling also causes the cut-off of the branches at a certain distance away from the outer periphery of the vessel (instead of tearing them off). This causes less blood loss and in most cases the vessel can still be used for bypasses.

When the distal tube end reaches the widening portion of the rod, the vein is cut off locally by pushing the sharp edge of the hollow tube against the olive shaped widening. The vein, the rod and the tube can then be removed via the groin without surrounding tissue disturbance and without damage, as experienced with the former described stripping techniques. The curl, located at the proximal end of the rigid rod, can be used to remove small side branches of the vein (VSM) in the groin by pushing the curl in the side branch and back, hooking up the branch, so these side branches can completely be removed via the groin.

### BRIEF DESCRIPTION OF THE DRAWINGS

Reference is made to the accompanying drawings, in which numerals are used to define parts of the device according to the invention and in which different embodiments of the invention are shown. The drawings representing respectively:
Figure 1 : a frontal view of a limb containing the vessel (VSM) that has to be removed.
Figure 2 : a rod or catheter according to the invention;
Figure 2A : a cross-sectional view along the line A-A in figure 2;
Figure 3 : a front view, partially cut-out, of a tube according to the invention;
Figure 3A : a cross-sectional view along the line A-A in figure 3;
Figure 4 : a cross-sectional partial view of the distal end of the tube;
Figure 4A : a front view of the tube according to the figure 4.
Figure 5 : a front view of an alternative embodiment (with three fingers) of the tube according to the invention;
Figure 6 : a cross-sectional partial view of another embodiment (with four fingers) of the distal end of the tube according to the invention;
Figure 6A : a front view of the embodiment shown in figure 6;
Figure 7 : an elevation view of another embodiment of the invention, showing only one protruding finger;
Figure 7A : a detailed sectional view of the extremity of one finger;
Figure 7B : a detailed sectional view of one part of a second tube in non active position;
Figure 7C : a detailed sectional view of one part of the second tube in active position;
Figure 8 : a front view of the embodiment according to figure 7.

### DETAILED DESCRIPTION

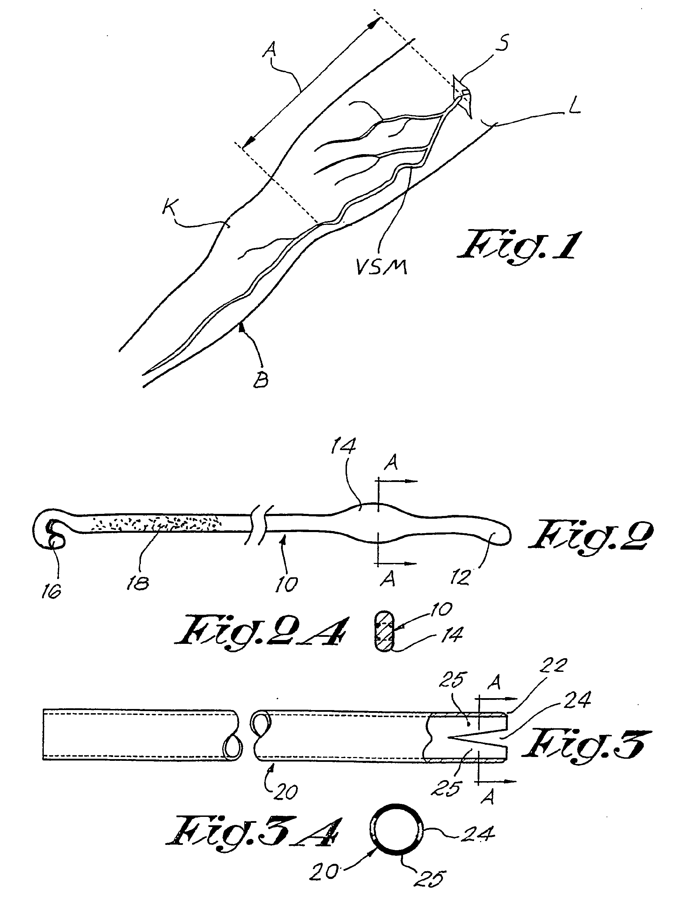

Figure 1 shows a leg B, in which a part of the vein VSM, over a distance A, between the groin and the knee has to be removed. At the level of the groin L, a small incision S is made to dissect the (cross of) the VSM and to open the proximal end of the vein so the rod or catheter, according to the invention, can be introduced.

As seen in figure 2 the first part of the device, according to the invention, consists of a rather rigid straight rod 10, from which only the distal end 12 and proximal end 16 are shown. The proximal end 16 of the rod 10 is provided with a corkscrew like curl. On a section, adjacent to the proximal end 16, the rod 10 is provided with a roughened surface 18.

The distal end 12 of the rod 10 is provided with a rounded, slightly bended, non traumatic shaped extremity. Close to its extremity, the distal end 12 of the rod 10 is provided with a substantially round or oval shaped broadened portion 14. This broadened portion or widening has a maximum dimension approximately equal to the inner diameter of the tube. The slightly bended and non traumatic distal end 12 of the rod is needed to assure accurate rod steering, in order to avoid damaging the inner side of the vessel. In case the distal part ends up in a side branch it is sufficient to pull back and to rotate the rod to avoid the side branch.

The proximal end of the rod 10 is provided with a curl 16 to facilitate the extraction of the side branches via the groin. Near to the curl 16, the proximal end the rod 10 is preferably made rough over a certain distance 18 to facilitate the fixation of the proximal end of the vessel VSM by means of a ligature.

To give a better idea of the shape and the dimensions of the rod 10, an example of the device is described hereafter. The rod can be made out of plastic, metal or any other suitable material. The rod has preferably a diameter of 2 mm and is about 75 cm long. The oval or round shaped broadened portion or widening is maximum 5 mm, the length 10 mm and the thickness 2 mm. The thickness corresponds with the diameter of the rod 10.

The at least second part of the device according to the invention is shown in figure 3. It consists of a hollow, rather flexible tube 20, which is generally provided with a sharp distal end 22.

According to the invention, the distal end 22 is provided with at least one, preferably two to four, longitudinal V-shaped slots 24, leaving between them, equally spaced, conical fingers 25 extending towards the distal end of the tube 20.

The slots 24 will be needed to locate and subsequently cut off the side branches of the vein VSM at a certain distance of its outer surface.

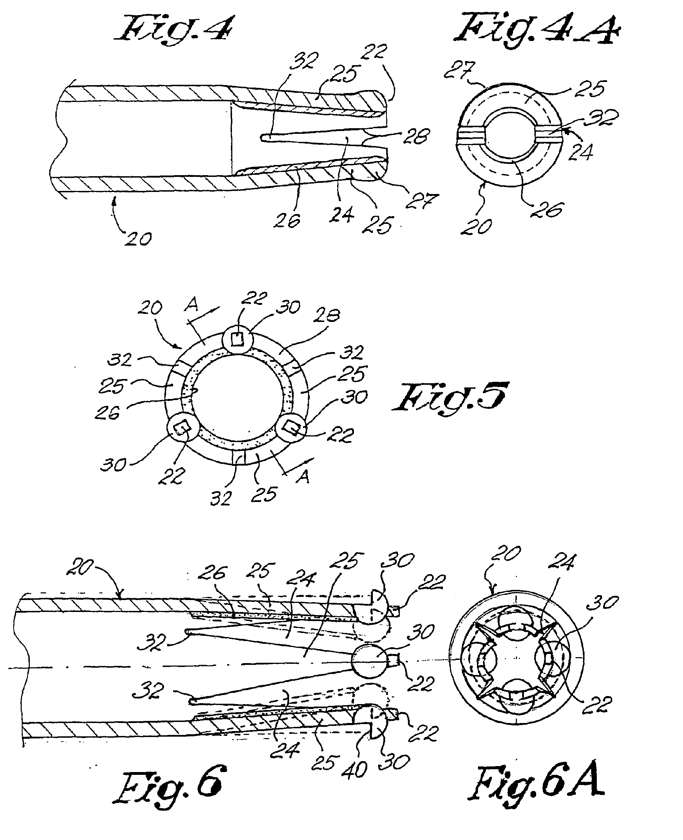

In a preferred embodiment shown in figure 4, said slots 24 are provided with a rear end in the shape of a gully 32, in which the side branches of the slot 24 remain parallel up to a rounded bottom end. The fingers 25 are slightly conical so that the extremity of the fingers 25 are positioned closer to the center of the tube 20. Preferably the extremities of the fingers 25 are provided with an enlarged rounded shape 27 so as to sufficiently remove the surrounding tissues from the outer surface of the vessel VSM.

In order to avoid any damage to the vein, which has to be isolated and removed from the human body, it is preferable to provide the distal end 22 of the tube 20 with an internal sleeve 26, as shown in figure.4A. This sleeve 26 is made out of a soft material so that it can smoothly push the external wall of the vein in a direction radial to the inside of the vein while pushing the blood out of the vein.

In any event, the fingers 25 of the distal end 22 of the tube 20 are mainly flexible due to the more or less deep slots 24, which is also advantageous for maintaining the vessel to be removed in the best possible condition for reuse.

According to other possible embodiments, the distal end 22 of the tube 20 can be realized as shown in figure 5 or 6.

Figure 5 shows a distal end 22 of the tube 20 provided with three equally spaced fingers 25. Between the fingers 25 are located the slots 24 ending up with respective gullies 32.

The head of the tube 20 here is shaped like a crown with oblique edges and the so formed distal ends are provided with spherical bullets 30. The extremities of the distal ends 22 are slightly protruding the periphery of the spherical bullets. These bullets 30 have a diameter slightly larger than the diameter of the wall of the tube 20. In that way they push away the surrounding tissue from the outer surface of the vein to the outside and, on the inside, they push the vein to the central part of the tube 20 where it is guided by the soft sleeve 26.

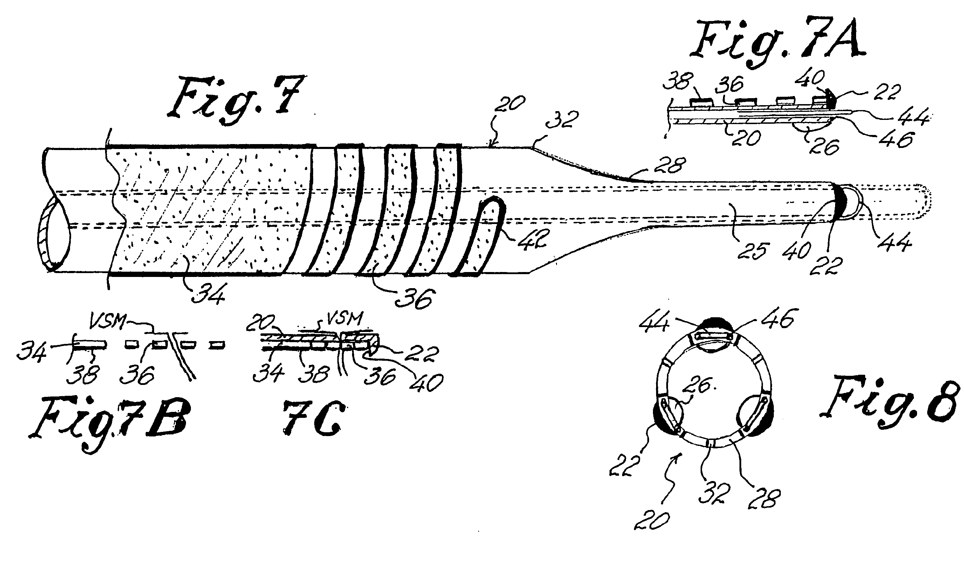

Figure 6 and 6A show a distal end of the tube 20 provided with four equally spaced fingers 25 which are extending slightly towards the center of the tube 20 and which are also provided with substantially spherical bullets 30 near their extremity 22. These bullets 30 are presenting an abutment shoulder 40, the purpose of which will be explained with the embodiment shown in figure 7.

Such head of the distal end of the tube 20 warrants that the vein is not damaged during the introduction of the tube 20 between the vein and the surrounding tissue.

Several fittings or attachments could be added to the device according to the invention such as for example a mechanism and/or device capable to cut off and/or to coagulate the side branches of the vein which are collected at the backside gully 32 of the slots 24. An example of such mechanism will be described hereafter.

As shown in the figure 7 to 8, the second part of the device according to the invention consisting of a tube (20) is provided with a finger (25) protruding towards the distal end (22). For clarity reasons, only one finger is shown on figure 7. These finger (25) is linked to the tube (20) by means of the side walls (28) of the slots (24) ending up in the gully (32).

According to this further embodiment of the invention, the additional fitting consists of a second tube 34, provided with a front end in the shape of an helicoidal spiral 36. Said tube 34 has a diameter slightly larger than the diameter of the first tube 20 so that it can slide smoothly over the outer surface of the tube 20. Preferably, said tube is made in material which is conductive for electrical power and is consequently provided with an isolating shield 38 to avoid contact with the surrounding tissues.

As shown in the detailed sectional view of figures 7A, 7B and 7C, the side branches of the vein VSM, which are collected into the gully 32 of the slot 24, will be positioned within the spiral 36 of the second tube 34 after this one has been pushed over the first tube 20 with a rotative movement (figure 7 B). The second tube 34 is pushed further towards the distal end 22 of the tube 20 and the front end 42 of the spiral 36 will abut against a rim or shoulder 40 provided on the bullet shaped end portions 22 of the first tube 20 (figure 7A).

By pushing the second tube 34 still further, the spiral 36 will be closed together (figure 7C) and the side branch of the vein VSM will be cut off and coagulated by means of electrical current applied to the second tube 34.

Instead of using a second tube 34 with a spiral end portion 36, one could also use a elongated spring element which could be introduced between the surrounding tissue and the first tube 20 and which could provide the same effect as the spiral end portion 36 to coagulate and cut off the side branches of the vein VSM due to the action of compression of the spring elements.

In order to improve further the separation of the vein VSM from the surrounding tissues, the fingers are provided with a flexible wire arrangement which can be manually operated from the back end of the first tube 20. As can be seen on the figures 7, 7A and 8, such a wire 44 is protruding from the extremity 22 of the finger 25 and is located in small channels 46 provided in the wall of the tube 20 ending up at each finger 25. By pushing the wire 44 at the rear side of and into the tube 20, the wire 44 will protrude further from the extremity of the distal end 22 as shown in dotted lines on figure 7.

Said distal end 22 is also provided, as explained before, with an interior bullet shaped sleeve 26 in soft material to push the wall of the vein VSM inwardly and to evacuate the venous blood.

The device according to the present invention allows the following general procedure :
- after dissection of the venous cross in the groin L, the first part of the invention, the rod 10, is introduced in the VSM and pushed further into the vein as far as the level of the knee. The bended edge 12 of the rod 10 makes it possible to pass along the valves and to avoid the side branches of the vein;
- in the groin L the VSM is tight up to the rough part of the rod 10 so that the combination, VSM and the rod 10, are fixed well to each other and none of them can move with regard to each other;
- the tube 20, with the sharp edge of the distal end 22 first, is then pushed over the VSM having the rod 10 on the inside. Because of the firm fixation of the VSM vein wall on the rod 10, in addition with a backward traction on the rod, the vein VSM is nicely aligned and the tube 20 can follow the vein easily;
- this action is performed with a slight pressure foreward and an alternative rotation movement with the tube 20 over the VSM in the direction of the knee;
- the surrounding tissue is dissected and separated from the outer periphery of the vein VSM and eventual side branches are cut off by the slots 24 at the distal end 22 of the tube 20. A further device improvement, according to the invention, consists that these side branches are not cut off at the level of their connection with the vein VSM and they are also not torn off from the VSM because they are only cut after they sustained the pressure of the tube 20 over a few centimeters. Due to the elasticity of the side branches of the vein, this pressure elongates the side branches before cutting them off.
- at the moment the distal end 22 of the tube 20 reaches the olive shaped widening 14 of the rod 10 the progression of the tube 20 stops. This distance, which corresponds with the length A of the VSM that has to be removed, can be marked beforehand on both parts 10 and 20 of the device;
- by pushing the hollow tube 20 vigorously with its sharp edge 22 against the olive shaped widening the vein is cut at the distal side at the level of the knee, at this moment the vein VSM is completely isolated in the tube 20;
- immediately after the evacuation out of the body, of both parts 10, 20 of the device together with the vein VSM, a compression bandage is applied over the remaining distal part of the VSM and the empty tunnel of the VSM to minimize bleeding.

Due to this procedure, and with the device as described above, the proximal part of the VSM can be removed, in case of varices, without an incision at the level of the knee K.

The VSM will not be torn off because there is no traction on it as in case of a classic stripping procedure.

For the same reason this device can be used under local anesthesia. Bleeding in the remaining tunnel is avoided mainly by application of a compression bandage and by the fact that the side branches are cut at a few centimeters from their connection with the VSM and are not torn as in case of a classic stripping technique.

Another important advantage of the device according to the invention is that the vein, removed from the body with this device, can be reused in case of cardiac or peripheral vascular bypass procedures.

The device according to the invention can, of course, be manufactured in different ways and is not limited to the embodiments as described above. The device can even be used for harvesting of venous material in case of a cardiac or peripheral vascular bypass procedure.

It is, for example, also possible to dissect the VSM first in the groin and then at the level of the knee via a second incision. A rod, similar to the rod 10, is introduced into the vein at the level of the knee and is proceeded in the direction of the groin.

By proceeding in the opposite direction, harm to the inside of the VSM and particularly tearing a valve is avoided.

In this design, the rod has no oval shaped widening but only a rough part at the proximal surface to attach the vein to the rod.

The rod is caught in the wound in the groin L and attached to the proximal end of the VSM.

The tube 20 is now carefully introduced into the vein VSM from the groin with a slight pressure and rotation so that the surrounding tissue is dissected and the side branches are cut off.

Essentially this procedure can be used for any part of the VSM because two incisions are made.

As a consequence, by means of the device according to the invention, any part of the vein in the subcutaneous tissue can be removed without damage so it can be reused in cardiac surgery as well as peripheral vascular surgery.

Further possible variations and extensions to the device are possible:
- The rod 10 can, at the distal end 12 between the bended part and the oval shaped widening, be loaded with a haemostatic plug; this plug can be delivered to the distal part of the VSM by manual pressure on the skin to avoid bleeding.
- As an alternative for this haemostatic plug the distal end of the tube 20 can be loaded at the distal end 20 with an elastic ring (silicone) that is pushed over the distal vessel to assure haemostasis of the distal VSM.
- The device can even be provided with a coagulation device at the distal end of the tube 20. The side branches of the VSM that arrive in the clefts 24 are not cut then but coagulated by means of an electrical rod that coagulates the side branches.
- To protect the vein even more in view of a possible reuse, a soft sleeve is fixated on the inside of the distal tip of tube 20 so that the wall of the VSM is first slightly removed from the cutting parts of the tube in the cleft 24 as well as on the distal tip 22.
- Instead of pushing the tube 20 over the VSM manually, a vibrating device can be attached to cause less damage to the vein and the surrounding tissue.
- The slots 24 of the tube 20 can for instance also be worked out helicoidally so that the side branches are cut at an even longer distance from the outer wall of the vein VSM.
- The device, according to the invention, obviously offers some important advantages with respect to the present existing state of the art technique. Nevertheless the device is not restricted to the description mentioned above and transformations can be performed without departing from the scope of the claims.

## Claims

1. A device for the removal of a blood vessel out of the human body in which the vessel is surrounded by tissues including the feature that the device mainly consists of two distinctive parts, a first part consisting of a rod capable of being introduced inside the vessel (VSM) over a certain length (A) and a second part, consisting of a tube, which has at least a length (A) and which can be pushed over the vessel (VSM) in order to isolate and dissect this vessel completely from the surrounding tissue,
**characterised in that**
the first part comprises a rod (10) having a distal end (12) which is slightly bent and non traumatically (round) shaped and **in that**
the second part comprises a tube (20) provided, on one extremity, with at least two longitudinal slots (24), leaving between them portions in the shape of fingers (25) extending towards the extremity (22) of the tube (20).

2. Device according to claim 1, including the feature that the bending end (12) of the rod (10) is provided with a broadening portion (14) located in proximity of said bending end (12).

3. Device according to claim 2, including the feature that the broadening portion (14) of the rod (10) is oval shaped.

4. Device according to claims 1 to 3, including the feature that the end of the rod 10), opposite to the bending end (12), is provided with a corkscrew like curl (16) and an adjacent section (18) having a roughened surface (18).

5. Device according to claim 1. including the feature that the rod (10) is made of rather stiff material.

6. Device according to claim 1. including the feature that the longitudinal slots (24) have sharp edges.

7. Device according to claims 1, including the feature that the longitudinal slots (24) are substantially V-shaped.

8. Device according to claim 1, including the feature that the fingers (25) of the tube (20) are provided with enlarged round shaped extremities (27, 30).

9. Device according to claim 1, including the feature that the tube (20) is provided at one end (22) with an internal sleeve (26) made of soft material.

10. Device according to claim 1 and 9, including the feature that the tube (20) is provided with a crown shaped end (22) consisting in oblique side walls (28) and finger (25) ends provided with spherical bullets (30) near the extremities of the crown shaped ends (22).

11. Device according to claim 10, including the feature that the diameter of the spherical bullets (30) is larger than the thickness of the wall of the tube (20).

12. Device according to claims 1 and 7, including the feature that the longitudinal slots (24) of the tube (20) is provided with a gully (32) to receive, cut and coagulate the side branches of the vein (VSM).

13. Device according to claim 1, including the feature that the extremities of the fingers (25) of the distal end (22) of the tube (20) are slightly inclined towards the center of the tube (20).

14. Device according to claims 1 and 13, including the feature that the maximum dimension of the broadening portion (14) of the rod is approximately equal to the inner diameter of the tube (20).

15. Device according to claim 1, including the feature that one extremity of the tube (20) is provided with at least two to four longitudinal slots (24), leaving between them portions in the shape of fingers (25) extending towards the extremity (22) of the tube (20) and an additional fitting, consisting of a second tube (34), presenting a front end in the shape of an helicoïdal spiral (36) and having a diameter slightly larger than the outer surface of the first tube (20) so that it can slide smoothly over said tube (20).

16. Device according to claim 1, including the feature that ohe extremity of said tube (20) is provided with at least two to four longitudinal slots (24) leaving between them portions in the shape of fingers (25) extending towards the extremity (22) of the tube (20), each finger being provided with a flexible wire arrangement consisting of a wire (44) protruding from the extremity (22) of the finger (25) and is free movable inside channels (46) located in the wall of the tube (20).

## Patentansprüche

1. Vorrichtung für die Entfernung eines Blutgefäßes aus dem menschlichen Körper, in welchem das Gefäß von Geweben umgeben ist, umfassend das Merkmal, dass die Vorrichtung hauptsächlich aus zwei unterschiedlichen Teilen besteht, nämlich einem ersten Teil, welcher eine Stange umfasst, die in das Gefäß (VSM) über eine bestimmte Länge (A) eingeführt werden kann, und einem zweiten Teil, welcher ein Rohr umfasst, welches wenigstens eine Länge (A) aufweist und welches über das Gefäß (VSM) geschoben werden kann, um dieses Gefäß zu isolieren und von dem umgebenden Gewebe vollständig zu trennen,
**dadurch gekennzeichnet, dass**
der erste Teil eine Stange (10) mit einem distalen Ende (12) umfasst, welches leicht gebogen ist und nicht traumatisierend (rund) geformt ist, und **dadurch**, dass der zweite Teil ein Rohr (20) umfasst, welches an einem Ende mit wenigstens zwei Längsschlitzen (24) versehen ist, die zwischen sich Teile in Fingerform (25) lassen, welche sich zu dem Ende (22) des Rohrs (20) erstrecken.

2. Vorrichtung nach Anspruch 1, umfassend das Merkmal, dass das gebogene Ende (12) der Stange (10) mit einem sich verbreiternden Teil (14) versehen ist, welcher in der Nähe des gebogenen Endes (12) angeordnet ist.

3. Vorrichtung nach Anspruch 2, umfassend das Merkmal, dass der sich verbreiternde Teil (14) der Stange (10) oval geformt ist.

4. Vorrichtung nach den Ansprüchen 1 bis 3, umfassend das Merkmal, dass das Ende der Stange (10) gegenüber dem gebogenen Ende (12) mit einer korkenzieherförmigen Windung (16) und einem benachbarten Abschnitt (18) mit einer angerauten Fläche (18) versehen ist.

5. Vorrichtung nach Anspruch 1, umfassend das Merkmal, dass die Stange (10) aus einem eher steifen Material gebildet ist.

6. Vorrichtung nach Anspruch 1, umfassend das Merkmal, dass die Längsschlitze (24) scharfe Kanten aufweisen.

7. Vorrichtung nach Anspruch 1, umfassend das Merkmal, dass die Längsschlitze (24) im Wesentlichen V-förmig sind.

8. Vorrichtung nach Anspruch 1, umfassend das Merkmal, dass die Finger (25) des Rohrs (20) mit vergrößerten, rund geformten Enden (27, 30) versehen sind.

9. Vorrichtung nach Anspruch 1, umfassend das Merkmal, dass das Rohr (20) an einem Ende (22) mit einer aus einem weichen Material gebildeten inneren Hülle (26) versehen ist.

10. Vorrichtung nach Anspruch 1 und 9, umfassend das Merkmal, dass das Rohr (20) mit einem kronenförmigen Ende (22) versehen ist, welches schräge Seitenwände (28) und Fingerenden (25) umfasst, die mit sphärischen Kugeln (30) in der Nähe der Enden der kronenförmigen Enden (22) versehen sind.

11. Vorrichtung nach Anspruch 10, umfassend das Merkmal, dass der Durchmesser der sphärischen Kugeln (30) größer ist als die Dicke der Wand des Rohrs (20).

12. Vorrichtung nach den Ansprüchen 1 und 7, umfassend das Merkmal, dass die Längsschlitze (24) des Rohrs (20) mit einer Rinne (32) versehen sind, um die Seitenzweige der Vene (VSM) aufzunehmen, zu schneiden und zu koagulieren.

13. Vorrichtung nach Anspruch 1, umfassend das Merkmal, dass die Enden der Finger (25) des distalen Endes (22) des Rohrs (20) leicht zur Mitte des Rohrs (20) hin geneigt sind.

14. Vorrichtung nach Anspruch 1 und 13, umfassend das Merkmal, dass die maximale Abmessung des sich verbreiternden Teils (14) der Stange ungefähr dem Innendurchmesser des Rohrs (20) entspricht.

15. Vorrichtung nach Anspruch 1, umfassend das Merkmal, dass ein Ende des Rohrs (20) mit wenigstens zwei bis vier Längsschlitzen (24) versehen ist, welche zwischen sich Teile in Fingerform (25) lassen, welche sich zu dem Ende (22) des Rohrs (20) erstrecken, sowie ein zusätzliches Anschlussstück, welches ein zweites Rohr (34) umfasst, das ein Vorderende in der Form einer helixförmigen Spirale (36) und einen Durchmesser aufweist, der geringfügig größer ist als die Außenfläche des ersten Rohrs (20), sodass es sanft über das Rohr (20) gleiten kann.

16. Vorrichtung nach Anspruch 1, umfassend das Merkmal, dass ein Ende des Rohrs (20) mit wenigstens zwei bis vier Längsschlitzen (24) versehen ist, welche zwischen sich Teile in Fingerform (25) lassen, welche sich zu dem Ende (22) des Rohrs (20) erstrecken, wobei jeder Finger mit einer flexiblen Drahtanordnung versehen ist, umfassend einen Draht (44), der von dem Ende (22) des Fingers (25) hervorsteht und in den in der Wand des Rohrs (20) angeordneten Kanälen (46) frei beweglich ist.

## Revendications

1. Dispositif permettant de retirer un vaisseau sanguin hors du corps humain, dans lequel le vaisseau est entouré par des tissus, comprenant la caractéristique que le dispositif est principalement constitué de deux parties distinctes, une première partie consistant en une tige qui peut être introduite à l'intérieur du vaisseau (VSM) sur une certaine longueur (A), et une deuxième partie consistant en un tube qui présente au moins une longueur (A) et qui peut être poussé sur le vaisseau (VSM) pour isoler et séparer ce vaisseau complètement par rapport aux tissus environnants,
**caractérisé en ce que**:
la première partie comprend une tige (10) présentant une extrémité distale (12) qui est légèrement pliée et de forme non traumatisante (arrondie), et la deuxième partie comprend un tube (20) comportant, à une extrémité, au moins deux fentes longitudinales (24) qui laissent entre elles des parties en forme de doigts (25) qui s'étendent en direction de l'extrémité (22) du tube (20).

2. Dispositif selon la revendication 1, comprenant la caractéristique que l'extrémité flexible (12) de la tige (10) présente une partie élargie (14) située à proximité de ladite extrémité flexible (12).

3. Dispositif selon la revendication 2, comprenant la caractéristique que la partie élargie (14) de la tige (10) est de forme ovale.

4. Dispositif selon les revendications 1 à 3, comprenant la caractéristique que l'extrémité de la tige (10), opposée à l'extrémité flexible (12), présente une boucle en forme de tire-bouchon (16) et une section adjacente (18) présentant une surface rugueuse (18).

5. Dispositif selon la revendication 1, comprenant la caractéristique que la tige (10) est constituée d'une matière assez rigide.

6. Dispositif selon la revendication 1, comprenant la caractéristique que les fentes longitudinales (24) ont des bords tranchants.

7. Dispositif selon la revendication 1, comprenant la caractéristique que les fentes longitudinales (24) sont sensiblement en forme de V.

8. Dispositif selon la revendication 1, comprenant la caractéristique que les doigts (25) du tube (20) présentent des extrémités rondes agrandies (27, 30).

9. Dispositif selon la revendication 1, comprenant la caractéristique que le tube (20) présente à une extrémité (22) un manchon interne (26) constitué d'une matière souple.

10. Dispositif selon les revendications 1 et 9, comprenant la caractéristique que le tube (20) présente une extrémité en forme de couronne (22) constituée de parois latérales obliques (28), et des extrémités de doigt (25) présentant des billes sphériques (30) à proximité des bouts des extrémités en forme de couronne (22).

11. Dispositif selon la revendication 10, comprenant la caractéristique que le diamètre des billes sphériques (30) est supérieur à l'épaisseur de la paroi du tube (20).

12. Dispositif selon les revendications 1 et 7, comprenant la caractéristique que les fentes longitudinales (24) du tube (20) comportent un couloir (32) pour recevoir, couper et coaguler les ramifications latérales de la veine (VSM).

13. Dispositif selon la revendication 1, comprenant la caractéristique que les extrémités des doigts (25) de l'extrémité distale (22) du tube (20) sont légèrement inclinées en direction du centre du tube (20).

14. Dispositif selon les revendications 1 et 13, comprenant la caractéristique que la dimension maximum de la partie élargie (14) de la tige est approximativement égale au diamètre intérieur du tube (20).

15. Dispositif selon la revendication 1, comprenant la caractéristique qu'une extrémité du tube (20) comporte au moins deux à quatre fentes longitudinales (24) laissant entre elles des parties en forme de doigts (25) qui s'étendent en direction de l'extrémité (22) du tube (20), et un raccord supplémentaire constitué d'un deuxième tube (34) présentant une extrémité avant en forme de spirale hélicoïdale (36) et ayant un diamètre légèrement supérieur à la surface extérieure du premier tube (20) de telle sorte qu'il puisse coulisser en douceur sur ledit tube (20).

16. Dispositif selon la revendication 1, comprenant la caractéristique qu'une extrémité dudit tube (20) comporte au moins deux à quatre fentes longitudinales (24) qui laissent entre elles des parties en forme de doigts (25) qui s'étendent en direction de l'extrémité (22) du tube (20), chaque doigt étant pourvu d'un arrangement de fil flexible constitué d'un fil (44) faisant saillie à partir de l'extrémité (22) du doigt (25) et pouvant se déplacer librement à l'intérieur de canaux (46) situés dans la paroi du tube (20).
